# EUROPEAN PATENT SPECIFICATION

(11) **EP 3 487 512 B1**
(45) Date of publication and mention of the grant of the patent: **31.12.2025**
(21) Application number: 17835086.4
(22) Date of filing: 25.07.2017
(51) Int. Cl.: A61K 35/32, A61K 38/39, A61K 47/30, A61L 27/22, A61L 27/12, A61K 47/69, A61L 27/54, C07K 7/08, C07K 7/06, C07K 5/10, C07K 5/02, A61P 19/08, A61K 9/14

(54) **PEPTIDE-COATED CALCIUM PHOSPHATE PARTICLES**
PEPTIDBESCHICHTETE CALCIUMPHOSPHATPARTIKEL
PARTICULES DE PHOSPHATE DE CALCIUM RECOUVERTES DE PEPTIDE

(30) Priority: 25.07.2016 US 201662366370 P
(43) Date of publication of application: 29.05.2019
(73) Proprietor: Cerapedics, Inc., Westminster, CO 80021 (US)
(72) Inventor: HANNIGAN, Nolan, Chase, Lakewood, CO 80232 (US); DAVIS, Katherine, Suzanne, Boulder, CO 80305 (US); BARNES, Tristan, Stuart, Louisville, CO 80027 (US); CONNOR, Jerome, Doylestown, PA 18902 (US)
(74) Representative: Kransell & Wennborg KB
(86) International application number: PCT/US2017/043614
(87) International publication number: WO 2018/022553

(56) References cited:
- WO-A2-2006/004778
- WO-A2-2011/005510
- WO-A2-2011/005510
- WO-A2-2013/096797

## Description

### Background of the Invention

The invention relates to the field of calcium phosphate particles coated with tightly bound peptides. The coated particles can be useful for repairing a variety of orthopedic conditions.

Physicians are sometimes called upon to repair bone that has been damaged by disease, trauma, osseous surgery or other causes, or to cause bone material to grow where there has been no bone before, such as during a spine fusion procedure. As an outcome of that procedure, it is desirable for two or more vertebral bodies to be maintained in a specific orientation. This can be accomplished by growing a column or bridge of rigid bone between the vertebral bodies. This maintains them in a fixed position relative to each other. The repair of long bone fractures can often be accomplished merely by relocating disrupted bone elements into natural proximity and fixing them in place until they can heal together. This is the approach taken in repairing ordinary limb fractures, for example. The fractured bone is re-set, then immobilized for a period of weeks in a rigid or semi-rigid cast or splint as the fractured elements heal.

Sometimes, however, this approach is insufficient because the patient has lost some of the bone. This can happen in certain kinds of trauma where the bone is so badly shattered that it cannot feasibly be pieced together. More often, it happens as a result of disease that destroys bone mass or as the result of osseous surgery in which destruction of bone mass is unavoidable. In these cases, there is no patient bone to re-set into proper position for healing. Instead, there is a void or defect that must somehow be filled, or a gap between two bone structures that needs to be filled with new bone. The filling of this defect or gap requires a material that is not only biocompatible but preferably will accept or even promote in-growing natural bone as the site heals. In such a manner, the material ideally will eventually become resorbed as new in-growing natural bone takes its place as part of the skeletal structure. Completely resorbed material eliminates the possibility for a stress riser that can occur when foreign matter remains in the skeleton, potentially giving rise to a fracture in the future.

Numerous bone replacement materials have been employed by physicians with varying degrees of success. One approach is to use bone material recovered from the patient himself, or so-called autologous bone. This approach is advantageous in that it avoids biocompatibility and bio-rejection problems. However, such an approach necessarily involves two surgical procedures, two surgical sites, and two healing processes--one for the original injury and a second for the site of the donated bone material. This means greater cost, and increased risk of infection and morbidity for a patient that is already seriously ill or injured. Also, this approach can require a great deal of time and surgical skill as the surgeon removes the donated material from the donation site, shapes and fits it to the primary site, and then repairs both sites. Finally, there is quite obviously a limit to the amount of bone in the patient's body available to be sacrificed as donor material.

Alternatively, a particulate bone graft substitute can be used to fill the bone defect. The selection of the particulate bone graft substitute depends upon its intended function in the treatment, its biocompatibility with the human body and its availability. A key limitation is whether the function of the treatment requires that the material be resorbed by natural bodily actions or remain in place as permanent supporting structures. Resorption of a particulate bone graft substitute (e.g., calcium phosphate particles) can be facilitation by coating the particles with cell adhesion peptides that function as an osteoconductive component, promoting bone deposition at the site of implantation.

WO 2011/005510 discloses compositions including a peptide conjugated to a binding group having affinity for a biocompatible calcified substrate, their use in implantable materials, and for the treatment of orthopedic conditions. There however remains a need for improved bone repair treatment formulations that include highly resorbable calcium phosphate particles.

### Summary of the Invention

The invention features methods and compositions for (i) controlling the amount of peptide bound to calcium phosphate particles, and (ii) for tightly binding peptide to the surface of calcium phosphate particles. The methods and compositions can be useful for promoting bone deposition at the site of implantation.

The invention features a composition including calcium phosphate particles coated with a cell adhesion peptide, wherein the cell adhesion peptide is tightly bound to the surface of the calcium phosphate particles and wherein the cell adhesion peptide is Gly-Thr-Pro-Gly-Pro-Gln-Gly-Ile-Ala-Gly-Gln-Arg-Gly-Val-Val (SEQ ID NO. 1, P-15 peptide), wherein the calcium phosphate particles are prepared by (i) providing pretreated calcium phosphate particles prepared by (a) combining the calcium phosphate particles with a first aqueous salt solution having an osmolarity value greater than 290 mOsm and a pH of between 6.5 and 8.5, (b) freezing said first aqueous salt solution, and (c) drying the calcium phosphate particles;(ii) combining the pretreated calcium phosphate particles with a second aqueous salt solution containing the cell adhesion peptide and having an osmolarity value greater than 290 mOsm and a pH of between 6.5 and 8.5 to produce coated calcium phosphate particles; and(iii) separating the coated calcium phosphate particles from the second aqueous salt solution,wherein the composition comprises P-15 peptide tightly bound to calcium phosphate particles such that following six, seven, or eight washes with a hypertonic buffer solution a concentration of greater than 100 ng of P-15 peptide per gram of the calcium phosphate particles remains bound to the calcium phosphate particles, and wherein, 8 weeks following implantation into a bone defect of sheep, the composition increases new bone formation in comparison to the implantation of P-15 peptide coated calcium phosphate particles not pretreated with saline or with freezing. In certain embodiments, the calcium phosphate particles are selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof. For example, the calcium phosphate particles can be hydroxyapatite particles. In particular embodiments, the amount of the cell adhesion peptide (e.g., P-15 peptide) tightly bound to the surface of the calcium phosphate particles is from 100 to 600 ng of cell adhesion peptide per gram of calcium phosphate particles (e.g., 100 to 600 ng of P-15 peptide per gram of inorganic bone mineral particles).

In a related aspect, the invention features a composition including hydroxyapatite particles coated with P-15 peptide, wherein the P-15 peptide is tightly bound to the surface of the hydroxyapatite particles and wherein the concentration of P-15 peptide bound to the surface of the hydroxyapatite particles is from 260 to 1,200 ng of P-15 per gram of hydroxyapatite particles (e.g., 300±40, 350±50, 400±50, 500±100, 600±100, 700±100, 800±100, or 1,000±200 ng of P-15 per gram of hydroxyapatite particles).

In one particular embodiment of the above aspects, the hydroxyapatite particles are inorganic bone mineral (ABM) particles.

In another particular embodiment of the above aspects, the particles have a mean particle size of from 100 µm to 1000 µm. For example, the particles can have a mean particle size of from 250 microns to 425 microns.

In another aspect, the invention features a method for coating calcium phosphate particles with a peptide, the method including: (i) combining the calcium phosphate particles with a first aqueous salt solution having an osmolarity value greater than 290 mOsm and a pH of between 6.5 and 8.5 (e.g., a pH of 6.8±0.3, 7.0±0.5, 7.5±0.5, or 8.0±0.5) to produce pretreated calcium phosphate particles; (ii) combining the pretreated calcium phosphate particles with a second aqueous salt solution containing the peptide and having an osmolarity value greater than 290 mOsm and a pH of between 6.5 and 8.5 (e.g., a pH of 6.8±0.3, 7.0±0.5, 7.5±0.5, or 8.0±0.5) to produce coated calcium phosphate particles; and (iii) separating the coated calcium phosphate particles from the second aqueous salt solution.

In yet another aspect, the invention features a method for coating calcium phosphate particles with P15 peptide, the method including: (i) providing pretreated calcium phosphate particles prepared by (a) combining the calcium phosphate particles with a first aqueous salt solution having an osmolarity value greater than 290 mOsm and a pH of between 6.5 and 8.5 and (b) freeze drying the calcium phosphate particles; (ii) combining the pretreated calcium phosphate particles with a second aqueous salt solution containing the peptide and having an osmolarity value greater than 290 mOsm and a pH of between 6.5 and 8.5 (e.g., a pH of 6.8±0.3, 7.0±0.5, 7.5±0.5, or 8.0±0.5) to produce coated calcium phosphate particles; and (iii) separating the coated calcium phosphate particles from the second aqueous salt solution.

In any of the above methods, the first aqueous salt solution or the second aqueous salt solution can include 4-(2-hydroxyethyl)-1-piperazineethanesulfonic acid (HEPES) buffer; N-Tris(hydroxymethyl)methyl-3-aminopropanesulfonic acid (TAPS) buffer; Diethanol glycine; Dihydroxyethylglycine (BHG, or Bicine) buffer; Tris(hydroxymethyl)aminomethane (Tris) buffer; N-(Tri(hydroxymethyl)methyl)glycine (Tricine) 3-[N-Tris(hydroxymethyl)methylamino]-2-hydroxypropanesulfonic acid (TAPSO) buffer; 2-[[1,3-dihydroxy-2-(hydroxymethyl)propan-2-yl]amino]ethanesulfonic acid (TES) buffer; 3-(N-morpholino)propanesulfonic acid (MOPS) buffer; piperazine-N,N'-bis(2-ethanesulfonic acid) (PIPES) buffer; or phosphate buffer. In one embodiment, the first aqueous salt solution or the second aqueous salt solution includes from 300 to 600 mM NaCl (e.g., 350±50, 400±50, 450±50, 500±50, or 550±50 mM NaCl). In another embodiment, the first aqueous salt solution or the second aqueous salt solution has an osmolarity value of between 400 and 1,200 mOsm (e.g., 500±100, 700±200, 900±200, or 1,100±100 mOsm). In particular embodiments of the methods, step (i) includes contacting the calcium phosphate particles with the first aqueous salt solution for a period of at least 2 hours (e.g., at least 2, 4, 8, 12, 24, or 48 hours). Step (i) can include combining from 5 to 15 mL of the first aqueous salt solution for each gram of the calcium phosphate particles. In particular embodiments of the methods, step (ii) includes contacting the pretreated calcium phosphate particles with the second aqueous salt solution for a period of at least 2 hours (e.g., at least 2, 4, 8, 12, 24, or 48 hours). Step (ii) can include combining from 1.5 to 5 mL of the second aqueous salt solution for each gram of the pretreated calcium phosphate particles. In another particular embodiments of the methods, step (iii) includes washing and drying the coated calcium phosphate particles. The washes can be performed with hypertonic buffer solution used to pretreat the particles in step (i). Six or more washes can be performed to produce calcium phosphate particle coated only with tightly bound peptide.

In particular embodiments, step (i) is free of the peptide used in step (ii) to coat the pretreated particles. In still other embodiments, step (i) includes agitating the particles during the pretreatment with the first aqueous solution (e.g., stirring, rolling, or mixing the particles and the hypertonic buffer for a period of 2, 4, 8, 12, 24, or 48 hours). In still other embodiments, the first aqueous solution differs from the second aqueous solution differ only in absence of the peptide to be coated onto the particles.

In any of the above methods, the calcium phosphate particles can be selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof. In particular embodiments, the calcium phosphate particles are hydroxyapatite particles (such as inorganic bone mineral particles). The particles can have a mean particle size of from 100 µm to 1000 µm. In certain embodiments, the particles have a mean particle size of from 250 microns to 425 microns.

In a further emodiment the method includes: (i) combining the calcium phosphate particles with an aqueous salt solution having an osmolarity value greater than 290 mOsm and a pH of between 6.5 and 8.5 (e.g., a pH of 6.8±0.3, 7.0±0.5, 7.5±0.5, or 8.0±0.5) to produce pretreated calcium phosphate particles; (ii) separating the calcium phosphate particles from the aqueous salt solution; and (iii) drying the calcium phosphate particles. The method can include, prior to step (iii), repeating steps (i) and (ii) two or more times (e.g., two, three, four, or five times). The aqueous salt solution can include HEPES buffer, TAPS buffer, Bicine buffer, Tris buffer, TAPSO buffer, TES buffer, MOPS buffer, PIPES buffer, or phosphate buffer. In one embodiment, the aqueous salt solution or the second aqueous salt solution includes from 300 to 600 mM NaCl (e.g., 350±50, 400±50, 450±50, 500±50, or 550±50 mM NaCl). In another embodiment, the aqueous salt solution has an osmolarity value of between 400 and 1,200 mOsm (e.g., 500±100, 700±200, 900±200, or 1,100±100 mOsm). In particular embodiments of the method, step (i) includes contacting the calcium phosphate particles with the aqueous salt solution for a period of at least 2 hours (e.g., at least 2, 4, 8, 12, 24, or 48 hours). Step (i) can include combining from 5 to 15 mL of the aqueous salt solution for each gram of the calcium phosphate particles (e.g., 4, 8, 12, 24, or 48 hours). In still other embodiments, step (i) includes agitating the particles during the pretreatment with the first aqueous solution (e.g., stirring, rolling, or mixing the particles and the hypertonic buffer for a period of 2, 4, 8, 12, 24, or 48 hours). The calcium phosphate particles can be selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof. In particular embodiments, the calcium phosphate particles are hydroxyapatite particles (such as inorganic bone mineral particles). The particles can have a mean particle size of from 100 µm to 1000 µm. In certain embodiments, the particles have a mean particle size **of** from 250 microns to 425 microns.

In a further embodiment the method step (i) includes (b) freezing the first aqueous salt solution, and (c) drying the calcium phosphate particles.

In the above methods, step (i) includes freeze drying the calcium phosphate particles.

In another embodiment of the above methods, step (i) includes freezing the first aqueous salt solution at ambient temperature in fewer than 30 minutes, 25 minutes, 20 minutes, 15 minutes, 10 minutes, or 5 minutes. In another embodiment of the above methods, step (i) includes freezing the first aqueous salt solution by cooling the first aqueous salt solution at a rate of at least 1 °C per minute, 2 °C per minute, or 3 °C per minute.

In still another embodiment of the above methods, step (i) includes freezing the first aqueous salt solution to a temperature of less than minus 5 °C, less than minus 7.5 °C, less than minus 10 °C, less than minus 15 °C, or less minus 20 °C.

In any of the above methods, the first aqueous salt solution or the second aqueous salt solution can include 4-(2-hydroxyethyl)-1-piperazineethanesulfonic acid (HEPES) buffer; N-Tris(hydroxymethyl)methyl-3-aminopropanesulfonic acid (TAPS) buffer; Diethanol glycine; Dihydroxyethylglycine (BHG, or Bicine) buffer; Tris(hydroxymethyl)aminomethane (Tris) buffer; N-(Tri(hydroxymethyl)methyl)glycine (Tricine) 3-[N-Tris(hydroxymethyl)methylamino]-2-hydroxypropanesulfonic acid (TAPSO) buffer; 2-[[1,3-dihydroxy-2-(hydroxymethyl)propan-2-yl]amino]ethanesulfonic acid (TES) buffer; 3-(N-morpholino)propanesulfonic acid (MOPS) buffer; piperazine-N,N'-bis(2-ethanesulfonic acid) (PIPES) buffer; or phosphate buffer. In one embodiment, the first aqueous salt solution or the second aqueous salt solution includes from 300 to 600 mM NaCl (e.g., 350±50, 400±50, 450±50, 500±50, or 550±50 mM NaCl). In another embodiment, the first aqueous salt solution or the second aqueous salt solution has an osmolarity value **of** between 400 and 1,200 mOsm (e.g., 500±100, 700±200, 900±200, or 1,100±100 mOsm). In particular embodiments of the methods, step (i) includes contacting the calcium phosphate particles with the first aqueous salt solution for a period of at least 2 hours (e.g., at least 2, 4, 8, 12, 24, or 48 hours). Step (i) can include combining from 5 to 15 mL of the first aqueous salt solution for each gram of the calcium phosphate particles. In particular embodiments of the methods, step (ii) includes contacting the pretreated calcium phosphate particles with the second aqueous salt solution for a period of at least 2 hours (e.g., at least 2, 4, 8, 12, 24, or 48 hours). Step (ii) can include combining from 1.5 to 5 mL of the second aqueous salt solution for each gram of the pretreated calcium phosphate particles. In another particular embodiments of the methods, step (iii) includes washing and drying the coated calcium phosphate particles. The washes can be performed with hypertonic buffer solution used to pretreat the particles in step (i). Six or more washes can be performed to produce calcium phosphate particle coated only with tightly bound peptide.

In particular embodiments, step (i) is free of the peptide used in step (ii) to coat the pretreated particles. In still other embodiments, step (i) includes agitating the particles during the pretreatment with the first aqueous solution (e.g., stirring, rolling, or mixing the particles and the hypertonic buffer for a period of 2, 4, 8, 12, 24, or 48 hours). In still other embodiments, the first aqueous solution differs from the second aqueous solution differ only in absence of the peptide to be coated onto the particles.

In any of the above methods, the calcium phosphate particles can be selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof. In particular embodiments, the calcium phosphate particles are hydroxyapatite particles (such as inorganic bone mineral particles). The particles can have a mean particle size of from 100 µm to 1000 µm. In certain embodiments, the particles have a mean particle size of from 250 microns to 425 microns.

In any of the above compositions, the composition includes P-15 peptide tightly bound to calcium phosphate particles at a concentration of greater than 100 ng of P-15 peptide per gram of the calcium phosphate particles (e.g., from 100 to 600 ng per gram of particles), and wherein, 8 weeks following implantation into a bone defect of sheep, the composition increases new bone formation in comparison to the implantation of calcium phosphate particles comprising less than 20 ng of P-15 peptide per gram of the calcium phosphate particles.

In any of the above compositions, the composition includes P-15 peptide tightly bound to calcium phosphate particles at a concentration of greater than 100 ng of P-15 peptide per gram of the calcium phosphate particles (e.g., from 100 to 600 ng per gram of particles). As described in Example 2, 8 weeks following implantation into a bone defect in sheep, the composition can increase new bone formation in comparison to the implantation of calcium phosphate particles not pretreated with saline or with freezing, or in comparison to calcium phosphate particles having less than 20 ng of P-15 peptide per gram of the calcium phosphate particles.

In another related aspect, the invention features an implant including calcium phosphate particles coated with a peptide, wherein said calcium phosphate particles are produced using any of the methods described herein, in combination with a hydrogel carrier (e.g., a carrier including carboxymethyl cellulose, collagen, or any other hydrogel-forming polymer described herein).

In yet another related aspect, the invention features a method of promoting the growth of bone in a subject by implanting calcium phosphate particles coated with a peptide, wherein said calcium phosphate particles are produced using any of the methods described herein, into the subject. The particles can be implanted, e.g., at the site of a bone defect, a bone void, or to promote spinal fixation of vertebral bodies (this method is not according to the invention as claimed).

As used herein, the term "cell adhesion peptide" refers to peptides of 3 to 100 amino acid residues in length (e.g., from 3 to 80, from 3 to 60, from 3 to 50, or from 3 to 40 amino acid residues in length) which are capable of binding to anchorage dependent cells via cell surface molecules, such as integrins, displayed on the surface of anchorage dependent cells.

As used herein, the term "tightly bound" refers to the portion of peptide retained on the surface of the calcium phosphate particles following six, seven, or eight washes with the hypertonic buffer solution. In the absence of the pretreatment of the calcium phosphate particles described herein, the remaining peptide bound to the surface following repeated washings is typically equal to or less than 20 ng of peptide per gram of calcium phosphate particles. Calcium phosphate particles bearing greater than 50 ng or 90 ng of peptide per gram of calcium phosphate particles tightly bound peptides can be prepared using the methods described herein. Using the methods of the invention, the calcium phosphate particles are pretreated with a hypertonic buffer solution prior to coating the particles with the desired peptide (e.g., an osteoconductive peptide, such as P-15 peptide). The pretreatment increases the amount of peptide bound to the surface of the particles and reduces the amount of peptide washed away from the surface when the particles are repeatedly washed with the hypertonic buffer solution.

Other features and advantages of the invention will be apparent from the following detailed description and the claims.

### Detailed Description

The invention features methods and compositions for (i) controlling the amount of peptide bound to calcium phosphate particles, and (ii) for tightly binding peptide to the surface of calcium phosphate particles. The methods and compositions can be useful for preparing implants useful for promoting bone deposition at the site of implantation, and for repairing a variety of orthopedic conditions. The invention features methods for pretreating calcium phosphate particles to increase the number of sites on the surface of the particles to which a peptide can tightly bind.

### Calcium phosphate particles

The formulations of the invention include a particulate calcium phosphate. The calcium phosphate may be any biocompatible, calcium phosphate material known in the art. The calcium phosphate material may be produced by any one of a variety of methods and using any suitable starting components. For example, the calcium phosphate material may include amorphous, apatitic calcium phosphate. Calcium phosphate material may be produced by solid-state acid-base reaction of crystalline calcium phosphate reactants to form crystalline hydroxyapatite solids. Other methods of making calcium phosphate materials are known in the art, some of which are described below. Alternatively, the calcium phosphate material can be crystalline hydroxyapatite (HA). Crystalline HA is described, for example, in U.S. Patent Nos. Re. 33,221 and Re. 33,161. These patents teach preparation of calcium phosphate remineralization compositions and of a finely crystalline, non-ceramic, gradually resorbable hydroxyapatite carrier material based on the same calcium phosphate composition. A similar calcium phosphate system, which consists of tetracalcium phosphate (TTCP) and monocalcium phosphate (MCP) or its monohydrate form (MCPM), is described in U.S. Patent Nos. 5,053,212 and 5,129,905. This calcium phosphate material is produced by solid-state acid-base reaction of crystalline calcium phosphate reactants to form crystalline hydroxyapatite solids. Carbonate substituted crystalline HA materials (commonly referred to as dahllite) may be prepared (see U.S. Patent No. 5,962,028). These HA materials (commonly referred to as carbonated hydroxyapatite) can be formed by combining the reactants with an aqueous liquid to provide a substantially uniform mixture, shaping the mixture as appropriate, and allowing the mixture to harden in the presence of water. During hardening, the mixture crystallizes into a solid and essentially monolithic apatitic structure. The reactants will generally include a phosphate source, e.g., phosphoric acid or phosphate salts, an alkali earth metal, particularly calcium, optionally crystalline nuclei, particularly hydroxyapatite or calcium phosphate crystals, calcium carbonate, and a physiologically acceptable lubricant. The dry ingredients may be pre-prepared as a mixture and subsequently combined with aqueous liquid ingredients under conditions where substantially uniform mixing occurs.

### Cell adhesion peptides

The calcium phosphate particles in the formulations of the invention can be coated with one or more cell adhesion peptides. Cell adhesion peptides can include any of the proteins of the extracellular matrix which are known to play a role in cell adhesion, including fibronectin, vitronectin, laminin, elastin, fibrinogen, and collagens, such as types I, II, and V, as well as their bioactive fragments. Additionally, the cell adhesion peptides may be any peptide derived from any of the aforementioned proteins, including derivatives or fragments containing the binding domains of the above-described molecules. Exemplary peptides include those having integrin-binding motifs, such as the RGD (arginine-glycine-aspartate) motif, the YIGSR (tyrosine-isoleucine-glycine-serine-arginine) motif, and related peptides that are functional equivalents. For example, peptides containing RGD sequences (e.g., GRGDS) and WQPPRARI sequences are known to direct spreading and migrational properties of endothelial cells (see V. Gauvreau et al., Bioconjug Chem. 16:1088 (2005)). REDV tetrapeptide has been shown to support endothelial cell adhesion but not that of smooth muscle cells, fibroblasts, or platelets, and YIGSR pentapeptide has been shown to promote epithelial cell attachment, but not platelet adhesion (see Boateng et al., Am. J. Physiol. Cell Physiol. 288:30 (2005). Other examples of cell-adhesive sequences are the NGR tripeptide, which binds to CD13 of endothelial cells (see L. Holle et al., Oncol. Rep. 11:613 (2004)) and DGEA that binds Type I collagen (see Hennessy et.al. Biomaterials, 30:1898 (2009)).

Cell adhesion peptides that can be used in the methods and compositions of the invention include, without limitation, those mentioned above, and the peptides disclosed in U.S. patent No. 6,156,572; U.S. patent publication No. 2003/0087111; and U.S. patent publication No. 2006/0067909,.

Alternatively, the cellular adhesion peptides can be obtained by screening peptide libraries for adhesion and selectivity to specific cell types (e.g. endothelial cells) or developed empirically via Phage display technologies.

In certain embodiments, the cell adhesion peptide is a collagen mimetic peptide. The integrin α2β1 consists of two non-identical subunits, α2 and βI, members of the integrin family each with a single trans-membrane domain, and α2β1 is known to bind to collagen via a specialized region of the α2-subunit. There are several known α2β1 recognition sites within collagens. This knowledge arises from the use of collagen fragments derived from purified α chains cleaved into specific and reproducible peptides. Collagen mimetic peptides that can be used in the implantable compositions of the invention include, without limitation, those described in PCT Publication Nos. WO/1999/050281; WO/2007/017671; and WO/2007/052067. Collagen mimetic peptides include, without limitation, peptides including the peptide sequences of any of SEQ ID NOS. 1-21: Gly-Thr-Pro-Gly-Pro-Gln-Gly-Ile-Ala-Gly-Gln-Arg-Gly-Val-Val (SEQ ID NO. 1, also known as "P-15"), Gly-Pro-Gln-Gly-Ile-Ala-Gly-Gln -Arg (SEQ ID NO: 2), Gln-Gly-Ile-Ala-Gly-Gln (SEQ ID NO: 3), Gln-Gly-Ile-Ala-Gly-Gln-Arg (SEQ ID NO: 4), Phe-Gly-Ile-Ala-Gly-Phe (SEQ ID NO: 5), Gly-Ile-Ala-Gly-Gln (SEQ IID NO: 6), Gln-Gly-Ala-Ile-Ala-Gln (SEQ ID NO: 7), Phe-Gly-Ile-Ala-Gly-Phe (SEQ ID NO:8), Cys-Gly-Ile-Ala-Gly-Cys (SEQ ID NO:9), Glu-Gly-Ile-Ala-Gly-Lys (SEQ ID NO:10), N-Acetyl Ile-Ala-Ala (SEQ ID NO:11), Ile-Ala-.beta.Ala (SEQ ID NO:12), N-Acetyl Ile-Ala NMe (SEQ ID NO:13), Asp-Gly-Glu-Ala (SEQ ID NO:14), Asp-Gly-Glu-Ala-Gly-Cys (SEQ ID NO:15), Gly-Phe-Pro*-Gly-Glu-Arg (SEQ ID NO:16, where Pro* = hydroxyproline), Gly-Leu-Pro*-Gly-Glu-Arg (SEQ ID NO:17, where Pro* = hydroxyproline), Gly-Met-Pro*-Gly-Glu-Arg (SEQ ID NO:18, where Pro* = hydroxyproline), Gly-Ala-Ser-Gly-Glu-Arg (SEQ ID NO:19), Gly-Leu-Ser-Gly-Glu-Arg (SEQ ID NO:20), Gly-Ala-Pro*-Gly-Glu-Arg (SEQ ID NO:21, where Pro* = hydroxyproline), and any other collagen mimetic peptides described in U.S. Patent No. 7,199,103.

For example, the cell adhesion peptide can be coated onto ABM particles have a mean particle diameter of 300 microns, and nearly all will fall within a range between 200 microns to 425 microns. However, a particle size range between 50 microns to 2000 microns may also be used.

As an alternative to inorganic bone mineral (ABM), a synthetic alloplast matrix or some other type of xenograft or allograft mineralized matrix that might not fit the definition of "inorganic" may be used in the methods and compositions of the invention as a substitute for calcium phosphate particles. The alloplast could be a calcium phosphate material or it could be one of several other inorganic materials that have been used previously in bone graft substitute formulations, e.g., calcium carbonates, calcium sulphates, calcium silicates, used in a mixture that includes calcium phosphate and that could function as biocompatible, osteoconductive matrices. The inorganic bone mineral, synthetic alloplast matrix, and xenograft or allograft mineralized matrix can be the particulate bone graft substitute and can be used to bind a cell adhesion peptide to their surface.

### Formulations

The invention features peptide-coated calcium phosphate particles which are useful for the treatment of bone defects. The coated particles can be incorporated into putties and lyophilized implants. Such implants can include, e.g., the coated particles suspended in a hydrogel carrier. Polysaccharides that may be utilized as a carrier include, for example, any suitable polysaccharide within the following classes of polysaccharides: celluloses/starch, chitin and chitosan, hyaluronic acid, alginates, carrageenans, agar, and agarose. Certain specific polysaccharides that can be used include agar methylcellulose, hydroxypropyl methylcellulose, carboxymethylcellulose, ethylcellulose, microcrystalline cellulose, oxidized cellulose, chitin, chitosan, alginic acid, sodium alginate, and xanthan gum.

The hydrogels will typically include a solvent to control the viscosity of the material. The solvent may be an alcohol or alcohol ester, including for example, glycerol, triacetin, isopropyl alcohol, ethanol, and ethylene glycol, or mixtures of these. The paste or gel can include others components, such as surfactants, stabilizers, pH buffers, and other additives (e.g., growth factors, antibiotics, analgesics, etc.). For example, a suitable gel or paste can be prepared using water, glycerin and sodium carboxymethylcellulose.

### Therapy

The compositions of the invention can be used in bone graft substitutes which are implanted into a subject. The compositions of the invention can include a cell adhesion peptide to promote rapid ossification of the implant.

The compositions of the invention can be useful for repairing a variety of orthopedic conditions. For example, the compositions may be injected into the vertebral body for prevention or treatment of spinal fractures, injected into long bone or flat bone fractures to augment the fracture repair or to stabilize the fractured fragments, or injected into intact osteoporotic bones to improve bone strength. The compositions can be useful in the augmentation of a bone-screw or bone-implant interface. Additionally, the compositions can be useful as bone filler in areas of the skeleton where bone may be deficient. Examples of situations where such deficiencies may exist include post-trauma with segmental bone loss, post-bone tumor surgery where bone has been excised, and after total joint arthroplasty (e.g., impaction grafting and so on). The compositions may be formulated as a paste prior to implantation to hold and fix artificial joint components in patients undergoing joint arthroplasty, as a strut to stabilize the anterior column of the spine after excision surgery, as a structural support for segmented bone (e.g., to assemble bone segments and support screws, external plates, and related internal fixation hardware), and as a bone graft substitute in spinal fusions.

The compositions of the invention can be used to coat prosthetic bone implants. For example, where the prosthetic bone implant has a porous surface, the composition may be applied to the surface to promote bone growth therein (i.e., bone ingrowth). The composition may also be applied to a prosthetic bone implant to enhance fixation within the bone.

The compositions of the invention can be used as a remodeling implant or prosthetic bone replacement, for example in orthopedic surgery, including hip revisions, replacement of bone loss, e.g. in traumatology, remodeling in maxillofacial surgery or filling periodontal defects and tooth extraction sockets, including ridge augmentation and sinus elevation. The compositions of the invention may thus be used for correcting any number of bone deficiencies at a bone repair site.

The following examples are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

### Example 1. Preparation of peptide-coated calcium phosphate particles.

The increased binding is achieved by pretreating, coating and washing the ABM in a buffer solution to equilibrate and stabilize the surface of the ABM particle before, during and after coating to increase the concentration of peptide tightly bound to the particles. The steps for coating the particles is described below.

Step 1: PRETREATMENT - ABM is pretreated in a Hypertonic (~460 mM NaCl) HEPES buffer, pH 7.2, at a 10:1 ratio (ml solution to g ABM) for 24 hours. This solution is decanted off after 24 hours. This wash is repeated with fresh solution for 1 hour or until there is no pH change upon exposure to fresh solution. The final wash solution is decanted and the ABM is vacuum dried. The pretreatment step is performed with agitation of some kind (roller, rotation, etc.).

Step 2: COATING - A P-15 coating buffer (concentration depends on final desired P-15 coated on ABM) is made using hypertonic HEPES, pH 7.2. Pretreated ABM is coated using a 2:1 ratio (mL solution to g ABM) for 24 hours. This step is performed statically, with no agitation.

Step 3: WASHING - After the 24 hour coating step, the solution is removed by a rapid vacuum filtration process. The coated ABM is washed 6-12 times in a 10:1 ratio of solution to ABM using hypertonic HEPES buffer, pH 7.2. Vacuum filtration is performed between each wash to remove the solution. After the final wash, the washed ABM is then vacuum dried overnight.

The pretreatment step equilibrates the surface of the ABM particles to a desired pH and osmolarity (ionic strength). The coating process is also performed under conditions similar to those used to pretreat the ABM to provide an improved coating environment for the peptide. Finally, the wash step can be performed using the same buffer to remove any extraneous peptide not bound to the ABM surface.

### Example 2. In vivo performance of P-15 Coated ABM Particles.

The performance of three test articles (a-c) were evaluated in a pre-clinical critical-defect, ovine drill hole model.

### Materials:

a) Control Article #1: uncoated ABM.
b) Test Article #1: P-15 coated ABM produced without pretreating the particles (< 20 ng-P-15 / g-ABM).
c) Test Article #2: ABM coated with P-15 using the pretreatment processing (ca. 250 ng-P-15 / g-ABM).

### Animal Model:

The Control and Test samples were implanted into a condyle site on the sheep femur. The drill hole was 8mm by 15mm is size. The samples were implanted for 4-weeks and 8-weeks. An empty defect was also included in the study. At the indicted end point the biopsies were harvested and prepared for histology. The amount of new bone formation generated by the samples was determined by histomophometry. In addition, a histological evaluation was performed to determine if any of the samples elicited an inflammatory response.

The uncoated ABM was delivered in a collagen carrier as a putty. The ABM with low P-15 concentration was delivered in a hydrogel carrier as a putty. The ABM with high P-15 was delivered in a collagen carrier as a putty.

The measurement of the inflammatory response was determined by the following scoring system:
0 = none present
1 = rare: 1-5 cells per high power field
2 = moderate: 6-10 cell per high power field
3 = heavy infiltration
4 = significant infiltration

The histomorphometric measurements quantitated the proportion of new bone formation within the original defect size as follows:
Total defect areas (TA)
New bone formation area (BA)
New bone formation: BA/TA

### Results:

Tables 1 and 2 display the histopathologist's review of the Test and Control samples at 4-weeks and 8-weeks with regards to new bone formation and infiltration of immune cells.

**Table 1. Histopathology & Histomorphometry Analysis - 4-weeks**

| Measurement | Empty Defect | ABM | ABM Low P-15 coating (< 20 ng-P-15 / g-ABM) | ABM High P-15 coating (ca. 250 ng-P-15 / g-ABM) |
|---|---|---|---|---|
| PMN | 1.00 ± 0.0 | 0.0 | 0.0 | 0.0 |
| Lymphocytes | 1.00 ± 0.0 | 0.0 | 0.0 | 0.0 |
| Macrophages | 2.00 ± 0.0 | 1.0 ± 0.0 | 1.25 ± 0.50 | 1.00 ± 0.0 |
| New Bone Formation | 26.8 ± 3.7 | 31.7 ± 7.3 | 47.3 ± 17.4 | 68.4 ± 13.4 |

**Table 2. Histopathology & Histomorphometry Analysis - 8-weeks**

| Measurement | Empty Defect | ABM | ABM Low P-15 coating (< 20 ng-P-15 / g-ABM) | ABM High P-15 coating (ca. 250 ng-P-15 / g-ABM) |
|---|---|---|---|---|
| PMN | 0.50 ± 0.71 | 0.0 | 0.0 | 0.0 |
| Lymphocytes | 0.0 | 0.0 | 0.0 | 0.0 |
| Macrophages | 1.00 ± 0.0 | 1.00 ± 0.0 | 1.00 ± 0.0 | 1.00 ± 0.0 |
| New Bone Formation | 48.4 ± 7.1 | 50.5 ± 9.3 | 63.1 ± 13.0 | 92.6 ± 4.4 |

### Conclusions:

The increased in concentration of P-15 bound to the ABM did not elicit any significant inflammatory response. Furthermore, the higher levels (>20 ng-P-15 / g-ABM) of P-15 tightly bound to the ABM yielded a significant increase in new bone formation as compared to the ABM coated with a low (< 20 ng-P-15 / g-ABM) P-15 concentration.

### Example 3. Effect of osmolarity on the binding of P-15 peptide to ABM particles.

ABM particles were incubated in a buffered solution that contained varying amounts of NaCl to modulate the final osmolarity. The ABM particles were incubated in the solutions for 18-24 hours, rinsed a second time in the same buffer, and dried. The pre-treated surface modified ABM particles were then incubated with P-15 dissolved in the same buffer as used during the pre-treatment step to coat the particles with P-15 peptide. Following the P-15 coating step, the ABM particles were washed repeatedly with the corresponding buffer solution. The amount of bound P-15 was determined using an ELISA test method. The results are provided in Table 3. The different runs represent different preparations of the same ABM source.

**Table 3. Effect of Osmolarity on P-15 Coating of ABM Granules**

| Buffer osmolarity (Hepes buffer, pH 7.2) | P-15 Concentration Bound to ABM Particles for Different ABM Sources (ng-P-15 / g-ABM) | | | | |
|---|---|---|---|---|---|
| | Bovine Cortical | | Bovine Cancellous | | Porcine Cancellous |
| | Run #1 | Run #2 | Run #1 | Run #2 | Run #1 |
| Hypotonic Solution (0% saline) | 105ng | 64ng | 68ng | 651ng | 231ng |
| Isotonic Solution (0.9% saline) | 116ng | 66ng | 129ng | 611ng | 283ng |
| Hypertonic Solution (2.7%saline) | 153ng | 105ng | 149ng | 1024ng | 376ng |

This data demonstrated that the treatment of ABM with a buffering solution that has a hypertonic saline concentration yields a higher level of bound P-15 peptide. This effect was applicable to all types of ABM (calcium phosphate) particles tested.

### Example 4. The effect of rate of freezing (rapid vs. slow freeze) on the amount of P-15 bound to the ABM particles.

Increased P-15 binding is achieved by freezing the pretreated ABM particles. The pretreated ABM particles are produced as described in step 1 of Example 1, where step 1 further includes freezing and then drying the ABM particles subjected to hypertonic buffer.

Paired samples of pretreated ABM particles were freeze-dried (F/D) by two different methods, and a third sample was simply air dried (without any freezing). One sample was placed on a controlled ambient temperature F/D shelf and a vacuum was immediately pulled to initiate rapid evaporative freezing (FD-EC). The second sample was placed on an ambient F/D shelf and slowly frozen by ramped freezing (~1-2 °C per minute). Once the sample was frozen the vacuum was initiated; this process is considered standard freeze-drying. The F/D process was used for both (a) the pretreatment step 1 and the washing (post-coating) step 3. The third samples was not frozen; the water was removed from the sample by airdrying at both drying steps. The following table indicates the amount of bound P15 on the final washed product. Table 4 indicates the amount of bound P-15 on the final washed ABM particles.

**Table 4. The effect of rate of freezing on the amount of P-15 bound to the ABM particles.**

| F/D Technique | P-15 ng/g ABM |
|---|---|
| Rapid freeze in freeze-dryer | 1521 |
| Slow freeze in freeze-dryer | 366 |
| Air drying | 74 |

### Conclusions:

A rapid rate of freezing, regardless of vacuum drying condition, resulted in higher amount of P-15 bound on the P-15 ABM particles. Simply drying the pretreated particles without freezing them (i.e., air drying) produced only modest improvements in the levels of P-15 binding compared to ABM not pretreated with hypertonic buffer.

### Example 5. The effect of rapid freeze in freeze-dry vs. intermediate freeze in vacuum oven on the amount of P-15 bound to the ABM particles.

Increased P-15 binding is achieved by rapidly freezing the pretreated ABM particles. The pretreated ABM particles are produced as described in step 1 of Example 1, where step 1 further includes freezing and then drying the ABM particles subjected to hypertonic buffer.

Paired samples of pretreated ABM particles were F/D by two different methods. One sample was placed on a controlled ambient temperature F/D shelf and a vacuum was immediately pulled to initiate rapid evaporative freezing. The second sample was placed on an uncooled shelf and a vacuum was immediately pulled to initiate evaporative cooling (vacuum oven drying). It should be noted that the rate of cooling is slower for this process due to the shelf temperature and the strength of vacuum. The F/D process was used for both (a) the pretreatment step 1 and the washing (post-coating) step 3. Table 5 indicates the amount of bound P-15 on the final washed ABM particles.

**Table 5. The effect of rapid freeze in freeze-drier vs. intermediate freeze in vacuum oven on the amount of P-15 bound to the ABM particles.**

| F/D Technique | P-15 ng/g ABM |
|---|---|
| Rapid freeze in freeze-dryer | 4201 |
| Intermediate freeze in vacuum oven | 308 |

### Conclusions:

A rapid rate of freezing, regardless of the use of evaporative cooling at the onset, resulted in higher amount of P-15 bound to the ABM particles.

### Example 6. The effect of freezing during the pretreatment step versus the P-15 coating step.

Increased P-15 binding is achieved by rapidly freezing the pretreated ABM particles relative to rapidly freezing only during the washing (post-coating) step 3.

Four paired samples of pretreated ABM particles were F/D by two methods, alternately performed at the two separate drying steps. Two samples were F/D by vacuum oven drying after the pretreatment step and then dried by either the vacuum oven drying process or by FD-EC (placed on a controlled ambient temperature F/D shelf and a vacuum was immediately pulled to initiate rapid evaporative freezing) method after the P-15 coating step. The other two samples were dried by FD-EC after the pretreatment step and then dried by either the vacuum oven drying process or by FD-EC method after the P-15 coating Table 6 table indicates the amount of bound P-15 on the final washed ABM particles.

**Table 6. FD-ED rapid drying procedure.**

| F/D Technique | | P-15 ng/g ABM |
|---|---|---|
| Pretreatment Step | P-15 Coating Step | |
| Vacuum oven | Vacuum oven | 72 |
| Vacuum oven | FD-EC | 77 |
| FD-EC | Vacuum oven | 261 |
| FD-EC | FD-EC | 351 |

### Conclusions:

The implementation of the FD-EC rapid freezing procedure only produced large changes in P-15 binding when applied to the ABM particles during the pretreatment step. While there was some decrease of the P-15 concentration when the slower freezing method was used following the coating step, effective and higher levels of P-15 coating were still achieved.

### Example 7. Blast-freeze followed by freeze-dryer vs. intermediate freeze in vacuum oven.

Increased P-15 binding is achieved by rapidly freezing the pretreated ABM particles. The pretreated ABM particles are produced as described in step 1 of Example 1, where step 1 further includes freezing and then drying the ABM particles subjected to hypertonic buffer.

Paired samples of pretreated ABM particles were F/D by two different methods. One sample was rapidly frozen using a blast-freezer. The frozen sample was placed on a cold F/D shelf and a vacuum was immediately pulled. The second samples was placed on an uncooled shelf and a vacuum was immediately pulled to initiate evaporative cooling. The F/D process was used for both (a) the pretreatment step 1 and the washing (post-coating) step 3. Table 7 indicates the amount of bound P-15 on the final washed ABM particles.

**Table 7. Blast-freeze followed by freeze-dryer vs. intermediate freeze in vacuum oven.**

| F/D Technique | P-15 ng/g ABM |
|---|---|
| Blast-freeze followed by standard freeze-dryer | 260 |
| Intermediate freeze in vacuum oven | 80 |

### Conclusions:

A rapid rate of freezing yielded a high concentration of P-15 bound to ABM and the rapid freezing at the pretreatment step can be achieved by multiple methods. The use of a blast-freezer to rapidly freeze the ABM worked similarly to the FD-EC method.

### Other Embodiments

While the invention has been described in connection with specific embodiments thereof, it will be understood that it is capable of further modifications and this application is intended to cover any variations, uses, or adaptations of the invention following, in general, including such departures from the present disclosure that come within known or customary practice within the art to which the invention pertains and follows in the scope of the claims.

## Claims

1. A composition comprising calcium phosphate particles coated with a cell adhesion peptide, wherein the cell adhesion peptide is tightly bound to the surface of the calcium phosphate particles and wherein the cell adhesion peptide is Gly-Thr-Pro-Gly-Pro-Gln-Gly-Ile-Ala-Gly-Gln-Arg-Gly-Val-Val (SEQ ID NO. 1, "P-15");
wherein the calcium phosphate particles are prepared by
(i) providing pretreated calcium phosphate particles prepared by (a) combining the calcium phosphate particles with a first aqueous salt solution having an osmolarity value greater than 290 mOsm and a pH of between 6.5 and 8.5, (b) freezing said first aqueous salt solution, and (c) drying the calcium phosphate particles;
(ii) combining the pretreated calcium phosphate particles with a second aqueous salt solution containing the cell adhesion peptide and having an osmolarity value greater than 290 mOsm and a pH of between 6.5 and 8.5 to produce coated calcium phosphate particles; and
(iii) separating the coated calcium phosphate particles from the second aqueous salt solution,
wherein the composition comprises P-15 peptide tightly bound to calcium phosphate particles such that following six, seven, or eight washes with a hypertonic buffer solution a concentration of greater than 100 ng of P-15 peptide per gram of the calcium phosphate particles remains bound to the calcium phosphate particles, and wherein, 8 weeks following implantation into a bone defect of sheep, the composition increases new bone formation in comparison to the implantation of P-15 peptide coated calcium phosphate particles not pretreated with saline or with freezing.

2. The composition of claim 1, wherein said calcium phosphate particles are selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof.

3. The composition of claim 2, wherein said calcium phosphate particles are hydroxyapatite particles.

4. A method for coating calcium phosphate particles with P-15 peptide, said method comprising:
(i) providing pretreated calcium phosphate particles prepared by (a) combining the calcium phosphate particles with a first aqueous salt solution having an osmolarity value greater than 290 mOsm and a pH of between 6.5 and 8.5 and (b) freeze drying the calcium phosphate particles;
(ii) combining the pretreated calcium phosphate particles with a second aqueous salt solution containing the peptide and having an osmolarity value greater than 290 mOsm and a pH of between 6.5 and 8.5 to produce coated calcium phosphate particles; and
(iii) separating the coated calcium phosphate particles from the second aqueous salt solution.

5. The method of claim 4, wherein the first aqueous salt solution or the second aqueous salt solution comprise HEPES buffer, TAPS buffer, Bicine buffer, Tris buffer, TAPSO buffer, TES buffer, MOPS buffer, PIPES buffer, or phosphate buffer.

6. The method of claim 4 or 5, wherein the first aqueous salt solution or the second aqueous salt solution comprises from 300 to 600 mM NaCl.

7. The method of any one of claim 4-6, wherein the first aqueous salt solution or the second aqueous salt solution has an osmolarity value of between 400 and 1,200 mOsm.

8. The method of any one of claims 4-7, wherein said calcium phosphate particles are selected from hydroxyapatite particles, dahllite particles, tetracalcium phosphate particles, calcium pyrophosphate particles, tricalcium phosphate particles, calcium hydrogen phosphate particles, octacalcium phosphate particles, calcium fluorapatite particles, and mixtures thereof.

9. The method of any one of claims 4-8, wherein said particles have a mean particle size of from 250 microns to 425 microns.

10. The method of any one of claims 4-9, said method comprising:
(i) providing pretreated calcium phosphate particles prepared by (a) combining the calcium phosphate particles with a first aqueous salt solution having an osmolarity value greater than 290 mOsm and a pH of between 6.5 and 8.5, (b) freezing said first aqueous salt solution, and (c) drying the calcium phosphate particles;
(ii) combining the pretreated calcium phosphate particles with a second aqueous salt solution containing the peptide and having an osmolarity value greater than 290 mOsm and a pH of between 6.5 and 8.5 to produce coated calcium phosphate particles; and
(iii) separating the coated calcium phosphate particles from the second aqueous salt solution.

11. The method of claim 10, wherein the method produces P-15 peptide tightly bound to calcium phosphate particles such that following six, seven, or eight washes with a hypertonic buffer solution a concentration of greater than 100 ng of P-15 peptide per gram of the calcium phosphate particles remains bound to the calcium phosphate particles, and wherein, 8 weeks following implantation into a bone defect of sheep, the composition increases new bone formation in comparison to the implantation of P-15 peptide coated calcium phosphate particles not pretreated with saline or with freezing.

## Patentansprüche

1. Zusammensetzung, welche Calciumphosphat-Partikel beschichtet mit einem Zelladhäsionspeptid umfasst, wobei das Zelladhäsionspeptid fest an die Oberfläche der Calciumphosphat-Partikel gebunden ist und wobei es sich bei dem Zelladhäsionspeptid um Gly-Thr-Pro-Gly-Pro-Gln-Gly-Ile-Ala-Gly-Gln-Arg-Gly-Val-Val (SEQ ID NR. 1, "P-15") handelt;
wobei die Calciumphosphat-Partikel hergestellt werden durch das
(i) Bereitstellen vorbehandelter Calciumphosphat-Partikel, hergestellt durch (a) das Kombinieren der Calciumphosphat-Partikel mit einer ersten wässrigen Salzlösung mit einem Osmolaritätswert über 290 mOsm und einem pH-Wert zwischen 6,5 und 8,5, (b) das Einfrieren der ersten wässrigen Salzlösung und (c) das Trocknen der Calciumphosphat-Partikel;
(ii) Kombinieren der vorbehandelten Calciumphosphat-Partikel mit einer zweiten wässrigen Salzlösung, welche das Zelladhäsionspeptid enthält und einen Osmolaritätswert über 290 mOsm und einen pH-Wert zwischen 6,5 und 8,5 aufweist, zum Erzeugen beschichteter Calciumphosphat-Partikel; und
(iii) Trennen der beschichteten Calciumphosphat-Partikel aus der zweiten wässrigen Salzlösung,
wobei die Zusammensetzung das P-15-Peptid fest an die Calciumphosphat-Partikel gebunden umfasst, sodass nach sechs, sieben oder acht Waschungen mit einer hypertonen Pufferlösung eine Konzentration von mehr als 100 ng P-15-Peptid pro Gramm der Calciumphosphat-Partikel an die Calciumphosphat-Partikel gebunden bleibt, und wobei die Zusammensetzung 8 Wochen nach Implantation in einen Knochendefekt bei Schafen die Knochenneubildung im Vergleich zur Implantation von mit P-15-Peptid beschichteten Calciumphosphat-Partikeln ohne Vorbehandlung mit Salzlösung oder Einfrieren erhöht.

2. Zusammensetzung nach Anspruch 1, wobei die Calciumphosphat-Partikel ausgewählt sind aus Hydroxyapatit-Partikeln, Dahllit-Partikeln, Tetracalciumphosphat-Partikeln, Calciumpyrophosphat-Partikeln, Tricalciumphosphat-Partikeln, Calciumhydrogenphosphat-Partikeln, Octacalciumphosphat-Partikeln, Calciumfluorapatit-Partikeln und Mischungen davon.

3. Zusammensetzung nach Anspruch 2, wobei es sich bei den Calciumphosphat-Partikeln um Hydroxyapatit-Partikel handelt.

4. Verfahren zur Beschichtung von Calciumphosphat-Partikeln mit P-15-Peptid, wobei das Verfahren Folgendes umfasst:
(i) Bereitstellen vorbehandelter Calciumphosphat-Partikel, hergestellt durch (a) das Kombinieren der Calciumphosphat-Partikel mit einer ersten wässrigen Salzlösung mit einem Osmolaritätswert über 290 mOsm und einem pH-Wert zwischen 6,5 und 8,5 und (b) das Gefriertrocknen der Calciumphosphat-Partikel;
(ii) Kombinieren der vorbehandelten Calciumphosphat-Partikel mit einer zweiten wässrigen Salzlösung, welche das Peptid enthält und einen Osmolaritätswert über 290 mOsm und einen pH-Wert zwischen 6,5 und 8,5 aufweist, zum Erzeugen beschichteter Calciumphosphat-Partikel; und
(iii) Trennen der beschichteten Calciumphosphat-Partikel aus der zweiten wässrigen Salzlösung.

5. Verfahren nach Anspruch 4, wobei die erste wässrige Salzlösung oder die zweite wässrige Salzlösung HEPES-Puffer, TAPS-Puffer, Bicin-Puffer, Tris-Puffer, TAPSO-Puffer, TES-Puffer, MOPS-Puffer, PIPES-Puffer oder Phosphatpuffer umfassen.

6. Verfahren nach Anspruch 4 oder 5, wobei die erste wässrige Salzlösung oder die zweite wässrige Salzlösung von 300 bis 600 mM NaCl umfassen.

7. Verfahren nach einem der Ansprüche 4-6, wobei die erste wässrige Salzlösung oder die zweite wässrige Salzlösung einen Osmolaritätswert zwischen 400 und 1.200 mOsm aufweist.

8. Verfahren nach einem der Ansprüche 4-7, wobei die Calciumphosphat-Partikel ausgewählt sind aus Hydroxyapatit-Partikeln, Dahllit-Partikeln, Tetracalciumphosphat-Partikeln, Calciumpyrophosphat-Partikeln, Tricalciumphosphat-Partikeln, Calciumhydrogenphosphat-Partikeln, Octacalciumphosphat-Partikeln, Calciumfluorapatit-Partikeln und Mischungen davon.

9. Verfahren nach einem der Ansprüche 4-8, wobei die Partikel eine mittlere Partikelgröße von 250 Mikronen bis 425 Mikronen aufweisen.

10. Verfahren nach einem der Ansprüche 4-9, wobei das Verfahren Folgendes umfasst:
(i) Bereitstellen vorbehandelter Calciumphosphat-Partikel, hergestellt durch (a) das Kombinieren der Calciumphosphat-Partikel mit einer ersten wässrigen Salzlösung mit einem Osmolaritätswert über 290 mOsm und einem pH-Wert zwischen 6,5 und 8,5, (b) das Einfrieren der ersten wässrigen Salzlösung und (c) das Trocknen der Calciumphosphat-Partikel;
(ii) Kombinieren der vorbehandelten Calciumphosphat-Partikel mit einer zweiten wässrigen Salzlösung, welche das Peptid enthält und einen Osmolaritätswert über 290 mOsm und einen pH-Wert zwischen 6,5 und 8,5 aufweist, zum Erzeugen beschichteter Calciumphosphat-Partikel; und
(iii) Trennen der beschichteten Calciumphosphat-Partikel aus der zweiten wässrigen Salzlösung.

11. Verfahren nach Anspruch 10, wobei das Verfahren das P-15-Peptid fest an die Calciumphosphat-Partikel gebunden erzeugt, sodass nach sechs, sieben oder acht Waschungen mit einer hypertonen Pufferlösung eine Konzentration von mehr als 100 ng P-15-Peptid pro Gramm der Calciumphosphat-Partikel an die Calciumphosphat-Partikel gebunden bleibt, und wobei die Zusammensetzung 8 Wochen nach Implantation in einen Knochendefekt bei Schafen die Knochenneubildung im Vergleich zur Implantation von mit P-15-Peptid beschichteten Calciumphosphat-Partikeln ohne Vorbehandlung mit Salzlösung oder Einfrieren erhöht.

## Revendications

1. Composition comprenant des particules de phosphate de calcium enrobées d'un peptide d'adhésion cellulaire, dans laquelle le peptide d'adhésion cellulaire est fortement lié à la surface des particules de phosphate de calcium et dans laquelle le peptide d'adhésion cellulaire est Gly-Thr-Pro-Gly-Pro-Gln-Gly-lle-Ala-Gly-Gln-Arg-Gly-Val-Val (SEQ ID NO. 1, « P-15 ») ;
dans laquelle les particules de phosphate de calcium sont préparées par
(i) la fourniture de particules de phosphate de calcium prétraitées préparées par (a) combinaison des particules de phosphate de calcium avec une première solution saline aqueuse ayant une valeur d'osmolarité supérieure à 290 mOsm et un pH compris entre 6,5 et 8,5, (b) congélation de ladite première solution saline aqueuse, et (c) séchage des particules de phosphate de calcium ;
(ii) la combinaison des particules de phosphate de calcium prétraitées avec une seconde solution saline aqueuse contenant le peptide d'adhésion cellulaire et ayant une valeur d'osmolarité supérieure à 290 mOsm et un pH compris entre 6,5 et 8,5 pour produire des particules de phosphate de calcium enrobées ; et
(iii) la séparation des particules de phosphate de calcium enrobées de la seconde solution saline aqueuse,
dans laquelle la composition comprend le peptide P-15 fortement lié à des particules de phosphate de calcium de telle sorte qu'après six, sept ou huit lavages avec une solution tampon hypertonique, une concentration supérieure à 100 ng de peptide P-15 par gramme des particules de phosphate de calcium reste liée aux particules de phosphate de calcium, et dans laquelle 8 semaines après l'implantation dans un défaut osseux de mouton, la composition augmente la formation de nouvel os par comparaison avec l'implantation de particules de phosphate de calcium enrobées de peptide P-15 non prétraitées avec une solution saline ou par congélation.

2. Composition selon la revendication 1, dans laquelle lesdites particules de phosphate de calcium sont choisies parmi les particules d'hydroxyapatite, les particules de dahllite, les particules de phosphate tétracalcique, les particules de pyrophosphate de calcium, les particules de phosphate tricalcique, les particules d'hydrogénophosphate de calcium, les particules de phosphate octacalcique, les particules de fluorapatite de calcium, et des mélanges de celles-ci.

3. Composition selon la revendication 2, dans laquelle lesdites particules de phosphate de calcium sont des particules d'hydroxyapatite.

4. Procédé d'enrobage de particules de phosphate de calcium avec le peptide P-15, ledit procédé comprenant :
(i) la fourniture de particules de phosphate de calcium prétraitées préparées par (a) combinaison des particules de phosphate de calcium avec une première solution saline aqueuse ayant une valeur d'osmolarité supérieure à 290 mOsm et un pH compris entre 6,5 et 8,5 et (b) lyophilisation des particules de phosphate de calcium ;
(ii) la combinaison des particules de phosphate de calcium prétraitées avec une seconde solution saline aqueuse contenant le peptide et ayant une valeur d'osmolarité supérieure à 290 mOsm et un pH compris entre 6,5 et 8,5 pour produire des particules de phosphate de calcium enrobées ; et
(iii) la séparation des particules de phosphate de calcium enrobées de la seconde solution saline aqueuse.

5. Procédé selon la revendication 4, dans lequel la première solution saline aqueuse ou la seconde solution saline aqueuse comprend un tampon HEPES, un tampon TAPS, un tampon Bicine, un tampon Tris, un tampon TAPSO, un tampon TES, un tampon MOPS, un tampon PIPES ou un tampon phosphate.

6. Procédé selon la revendication 4 ou 5, dans lequel la première solution saline aqueuse ou la seconde solution saline aqueuse comprend de 300 à 600 mM de NaCl.

7. Procédé selon l'une quelconque des revendications 4 à 6, dans lequel la première solution saline aqueuse ou la seconde solution saline aqueuse a une valeur d'osmolarité comprise entre 400 et 1 200 mOsm.

8. Procédé selon l'une quelconque des revendications 4 à 7, dans lequel lesdites particules de phosphate de calcium sont choisies parmi les particules d'hydroxyapatite, les particules de dahllite, les particules de phosphate tétracalcique, les particules de pyrophosphate de calcium, les particules de phosphate tricalcique, les particules d'hydrogénophosphate de calcium, les particules de phosphate octacalcique, les particules de fluorapatite de calcium, et des mélanges de celles-ci.

9. Procédé selon l'une quelconque des revendications 4 à 8, dans lequel lesdites particules ont une granulométrie moyenne de 250 micromètres à 425 micromètres.

10. Procédé selon l'une quelconque des revendications 4 à 9, ledit procédé comprenant :
(i) la fourniture de particules de phosphate de calcium prétraitées préparées par (a) combinaison des particules de phosphate de calcium avec une première solution saline aqueuse ayant une valeur d'osmolarité supérieure à 290 mOsm et un pH compris entre 6,5 et 8,5, (b) congélation de ladite première solution saline aqueuse, et (c) séchage des particules de phosphate de calcium ;
(ii) la combinaison des particules de phosphate de calcium prétraitées avec une seconde solution saline aqueuse contenant le peptide et ayant une valeur d'osmolarité supérieure à 290 mOsm et un pH compris entre 6,5 et 8,5 pour produire des particules de phosphate de calcium enrobées ; et
(iii) la séparation des particules de phosphate de calcium enrobées de la seconde solution saline aqueuse.

11. Procédé selon la revendication 10, dans lequel le procédé produit le peptide P-15 fortement lié à des particules de phosphate de calcium de telle sorte qu'après six, sept ou huit lavages avec une solution tampon hypertonique, une concentration supérieure à 100 ng de peptide P-15 par gramme des particules de phosphate de calcium reste liée aux particules de phosphate de calcium, et dans lequel 8 semaines après l'implantation dans un défaut osseux de mouton, la composition augmente la formation de nouvel os par comparaison avec l'implantation de particules de phosphate de calcium enrobées de peptide P-15 non prétraitées avec une solution saline ou par congélation.
